# EUROPEAN PATENT APPLICATION

(11) **EP 1 956 372 A1**
(43) Date of publication of application: **13.08.2008**
(21) Application number: 06756950.9
(22) Date of filing: 02.06.2006
(51) Int. Cl.: G01N 33/53, G01N 33/543, G01N 33/545, G01N 33/547

(54) **ULTRAHIGHLY SENSITIVE DETERMINATION REAGENT FOR C-REACTIVE PROTEIN AND DETERMINATION METHOD**

(30) Priority: 30.11.2005 JP 2005345713
(71) Applicant: Nihon University, Tokyo 102-8275 (JP)
(72) Inventor: KOHNO, Hideki, Tokyo 1028275 (JP); OGAWA, Masahiro, Tokyo 1028275 (JP)
(74) Representative: Hartz, Nikolai
(86) International application number: PCT/JP2006/311129
(87) International publication number: WO 2007/063616

(57) **Abstract**

The present invention is related to an ultrahighly sensitive determination reagent for C-reactive protein (CRP) for use in determination of CRP in a test sample with high sensitivity; and a CRP determination method using the reagent. A reagent for determining C-reactive protein containing an insoluble carrier particle loading an antibody directed to the C-reactive protein via an amino acid; and a method for determining a C-reactive protein including the steps of reacting the reagent with an antigenic substance in a test sample, and determining a resulting aggregate.

## Description

### Technical Field

The present invention relates to an ultrahighly sensitive C-reactive protein determination reagent for determining C-reactive protein (CRP) in a test sample with extremely high sensitivity, and a CRP determination method using the reagent.

### Background Art

A C-reactive protein (CRP) is a serum protein which precipitates in the presence of C-polysaccharides from *Diplococcus pneumoniae* and which generally exists in the serum of healthy human individuals in trace quantities (average 580 ng/ml). The CRP increases rapidly in blood in response to inflammatory diseases or tissue degeneration/necrosis and decreases rapidly as the disease state subsides. Thus the determination of blood CRP concentration finds wide clinical application in the determination of the severity, progress, prognosis or therapeutic efficacy for diseases such as bacterial infectious diseases, myocardial infarctions and malignant tumors (Refer to Non-Patent Document 1).
Since conventional methods of determining CRP determine a large increase from the normal concentration occurring during acute inflammation, extremely sensitive determination methods were not so much required. More recently, however, the development of highly sensitive methods of CRP determination such as latex agglutination tests using immunoassay reagents loading antibodies on latex particles has lead to the determination of CRP concentrations in low concentration ranges. Since determination of a CRP concentration in a low concentration range can be used as a diagnostic marker for myocardial infarction, neonatal infectious diseases and the like and relates to various diseases such as periodontosis, this has attracted considerable attention (Refer to Patent Document 1 and Non-Patent Document 2).

In a latex agglutination test, a CRP antibody-sensitized latex reagent binds to CRP antigen in a sample by an antigen-antibody reaction to form an aggregate. A CRP concentration is determined by detecting the aggregate. Highly sensitive determination at low concentration ranges using a small amount of an antigen is possible due to the formation of a large aggregate. Currently, however, since determination of CRP using latex agglutination tests used clinically is limited to concentrations of about 500 ng/ml, this method cannot be applied to localized inflammation or small lesions. Therefore there is a need for the detection of CRP at trace levels which can be applied to smaller inflammations and lesions than available using conventional methods. Consequently there is a need to further increase the sensitivity of CRP determination reagents.
[Patent Document 1] JP-A-2001-330615
[Non-Patent Document 1] Yoshio Fukuoka et al. Clinical Immunology, Ishiyaku Publishers,Inc., 1997.
[Non-Patent Document 2] Hakuo Takahashi "Application of Highly Sensitive CRP Determination Method to Pathological Diagnosis", Clinical Pathology, 2002, Vol. 50, pp 30-39.

### Disclosure of the Invention

### Problems to be Solved by the Invention

An object of the present invention is to provide measures for determining CRP in a test sample with high sensitivity and in a simple and rapid manner.

### Means for Solving the Problems

The inventors reached the present invention by preparing a latex reagent sensitized to an anti-CRP antibody by chemically modifying the surface of the latex particle to allow the introduction of a spacer molecule. Upon testing the increase in sensitivity, high sensitivity was not obtained when using a spacer such as a polypeptide (polyglutamic acid) disclosed in Patent Document 1 since the antibody could not be stably fixed in a constant orientation. However, when an amino acid was used as the spacer molecule to load the anti-CRP antibody on the latex particle via the amino acid, antibody could be stably fixed in a constant orientation. Thus the present invention enables the determination of CRP present in a test sample with extremely high sensitivity.

In other words, the present invention provides a C-reactive protein determination reagent comprising an insoluble carrier particle loading an antibody directed to the C-reactive protein via an amino acid.
Furthermore the present invention provides a method of determining a C-reactive protein comprising the steps of reacting the determination reagent with an antigenic substance in a test sample, and determining the resulting aggregate.

### Effects of the Invention

The CRP determination reagent of the present invention can detect CRP in a test sample with extremely high sensitivity and thus can be applied to extremely small internal inflammations such as localized inflammation or small lesions and is extremely useful in laboratory tests for distinguishing a patient from a healthy individual and for monitoring the course of treatment.

### Brief Description of the Drawings

Figure 1 shows the results of the reactivity of each latex reagent using an amino acid as a spacer molecule;
Figure 2 shows the results of the reactivity of a latex reagent using an amino acid as a spacer molecule, a latex reagent not using a spacer molecule and a latex reagent using protein A as a spacer molecule; and
Figure 3 shows the concentration in the blood of CRP in a patient suffering from liver disease.

### Best Mode for Carrying Out the Invention

The CRP determination reagent of the present invention loads an antibody directed to CRP on an insoluble carrier particle via an amino acid.
Any carrier may be used as the insoluble carrier particle of the present invention as long as it is insoluble, does not participate in non-specific reactions and is stable. Examples of the insoluble carrier particle include latex particles, bentonite, collodion, kaolin and fixed sheep erythrocyte, and latex particles are particularly preferred.

There is no particular limitation on the latex particle but it is preferred to use organic particulates having a comparatively fixed particle size and having constant quality and behavior. The use of organic particulates capable of industrial scale mass production is preferred. There is no particular limitation on the organic particulates and they include, for example, homopolymers and/or copolymers of vinyl monomers such as styrene, vinyl chloride, acrylonitrile, vinyl acetate, acrylic ester and methacrylic ester; and butadiene copolymers such as styrene-butadiene copolymers and methyl-methacrylatebutadiene copolymers. Of these, polystyrene latex particles are preferred in view of their long-term stable biological activity.
The latex particle may include a functional group on the particle surface. The functional group may have a feature capable of fixing an amino acid used as a molecular spacer and includes, for example, a carboxyl group, an amino group, an aldehyde group, a thiol group, a glycidyl group, an acyl halide group, an isocyanate group, an isothiocyanate group, active ester groups and acid anhydride groups. Of these, carboxyl-modified latex particles are preferable. Commercially available particles such as "Immutex" (product of JSR Corporation) can be used.

The latex particle preferably has a particle size of 0.01 to 1 µm. When the particle size is less than 0.01 µm, a plurality of minute aggregates of heterogeneous particle sizes are produced and have an adverse effect on simultaneous reproducibility. Furthermore there may not be sufficient aggregates for the number of antibodies.
On the other hand, when the particle size exceeds 1 µm, autoaggregation is promoted, resulting in a tendency for reduced dispersion.

The amino acid used in the present invention include an amino acid, a salt thereof and a corresponding hydrate thereof. The amino acid may be a L-amino acid or a D-amino acid or may be a racemic form. There is not particular limitation on the amino acid and examples thereof include any of the following: neutral amino acids (Gly, Ala, Ser, Thr, Cys, Cys-Cys, Asn, Gln, Leu, Ile, Val, Met, Phe, Tyr, Trp, Pro), acidic amino acids (Asp, Glu) and basic amino acids (His, Lys, Arg). There is no particular limitation on the salt of amino acid and examples thereof include salts of alkali metals such as sodium and potassium; salts of alkaline earth metals such as magnesium and calcium; salts of inorganic acids such as hydrochloric acid, sulfuric acid and phosphoric acid; and salts of organic acids such as acetic acid, citric acid and p-toluenesulfonic acid. From the point of view of reactivity, it is preferred to use basic amino acids (His, Lys, Arg), sulfur-containing amino acids (Cys, Met), oxyamino acids (Ser, Thr), aliphatic amino acids (Gly, Val, Leu), acidic amino acid (Asp, Glu), an acidic aminoacid amide (Gln), salts thereof and the hydrates thereof. In particular, the use of Arg and Gln are preferred due to the chemical nature of these amino acids.
It is preferred to fix a plurality of amino acids on a single insoluble carrier.

The antibody directed to CRP (anti-CRP antibody) of the present invention may specifically bind to CRP and there is no particular limitation on its origin, type (monoclonal or polyclonal) or configuration. There is no particular limitation on the origin of the antibody, which includes human, mouse, rat, rabbit, goat and monkey, etc. There is no particular limitation on the purity of the antibody and, for example, it may be in the form of a globulin fraction or an affinity purified fraction. The antibody may be prepared in accordance with known methods or may be commercially purchased.
The antibodies used in the present invention are not limited to whole antibody molecules. As long as the antibody binds to CRP, antibody fragments or modified antibody fragments may be used, including monovalent and bivalent antibodies. Examples of the antibody fragments include Fab, F(ab')₂, Fv, Fab/c with one Fab and a complete Fc, and single chain Fv (scFv) to which an H chain or an L chain from Fv is connected using a suitable linker.

In the present invention, there is no particular limitation on the method of binding the amino acid to the insoluble carrier or the method of binding the anti-CRP antibody to the amino acid as long as inseparable binding occurs. For example, this includes chemical bonding such as covalent bonding.

When using a carboxyl-modified latex particle as the insoluble particle, a part of the CRP determination reagent of the present invention is represented by the following formula (1) and may be prepared using a method described below:

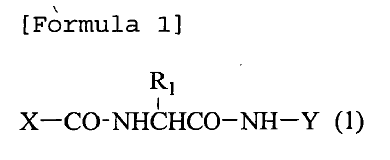

(wherein X-CO- denotes a residue originating from a carboxyl-modified latex particle, NHCH(R₁)CO- denotes a residue from an amino acid and NH-Y denotes a residue from an anti-CRP antibody).
That is to say, the amino group on the N-terminus of the amino acid and the carboxyl group on the surface of the latex particle undergo a dehydration-condensation reaction to form a peptide bond using a known method such as an activated ester method or a mixed anhydride method. Thereafter the latex reagent of the present invention may be obtained by adding an anti-CRP antibody solution and binding to the amino acid using a known coupling method.

There is no particular limitation on the method of condensing the carboxyl group on the surface of the latex particle and the amino group on the N-terminus of the amino acid. For example, 0.5 to 2.0% (w/v) of latex particles are suspended in buffer solution, a water soluble carbodiimide such as dicyclohexylcarbodiimide or N-ethyl-N'-(3-dimethylaminopropyl) carbodiimide (WSC), etc., and N-hydroxy-bromosuccinimide (NHS), N-hydroxybenzotriazole and the like are added. The mixture is reacted at a temperature between 0°C to a reflux temperature, preferably at room temperature, for 20 minutes to 1 hour in order to obtain active esterification of the carboxyl group. Amino acid at a concentration of 0.1 × 10⁻⁴ to 10 × 10⁻⁴ mol/mL, preferably 0.5 × 10⁻⁴ to 5 × 10⁻⁴ mol/mL, is then added, the mixture is heated to a temperature of between room temperature to near the boiling point of the solvent and left to react for 30 minutes to 2 hours. The buffer solution used in the reaction includes, for example, MES buffer solution, phosphate buffer solution, Tris-HCl buffer solution, citrate buffer solution, borate buffer solution and carbonate buffer solution. The pH during the reaction is between pH of 5 to 10, preferably a pH of 6 to 9.

After the carboxyl group on the C-terminus of the amino acid undergoes active esterification in the manner described above, the anti-CRP antibody is coupled to the amino acid loaded to the latex particle by adding 50 to 400 µg/ml of anti-CRP antibody to the buffer solution described above and allowing the mixture to react at 4°C to 40°C for 20 minutes to 1 hour. After the reaction, the latex reagent of the present invention can be obtained by normal additional treatments such as centrifugation and blocking.

In this manner, the CRP determination reagent of the present invention can retain an anti-CRP antibody in a stable and fixed orientation using an amino acid to load the anti-CRP antibody on the insoluble carrier. For example, a CRP determination method using a conventional LPIA method is limited to a sensitivity of about 500 ng/mL and has no application to localized inflammation or small lesions. However, the present invention is effective at sensitivities of 10 ng/mL which is substantially 50 times higher and can be used on extremely small internal inflammations. Thus the CRP determination reagent of the present invention finds application to diagnosis and treatment follow-up of diseases including various types of infectious diseases, inflammatory diseases and diseases involving tissue breakdown.
Examples of such diseases include inflammations such as rheumatism, viral hepatitis, pneumonia, macular degeneration and urinary tract infections, tissue-destroying diseases, communicable diseases, and deterioration in the inflammation of the tonsillitis palatina.

There is no particular limitation on the concentration of antibodies directed to CRP in the CRP determination reagent of the present invention as long as the concentration can determine the CRP in a test sample. A concentration of 50 to 400 µg/mL is preferred, and a concentration of 100 to 200 µg/mL is particularly preferred. Sensitivity is low at less than 50 µg/mL and accurate determination in such low concentration ranges is difficult whereas a concentration exceeding 400 µg/mL tends to result in non-specific aggregates.

In addition to the carrier loading the anti-CRP antibody, the CRP determination reagent of the present invention may, for example, also contain suitable amounts of bovine serum albumin or sucrose and the like dissolved for the purpose of the prevention of non-specific reactions and preservation stability. Furthermore sodium chloride may be dissolved therein in order to adjust the salt concentration.
The CRP determination reagent of the present invention may be provided as a single-pack containing a dispersed solution of the insoluble carrier loading an anti-CRP antibody via an amino acid, or it may be used in a two-pack or three-pack format. The reagent may include a kit, which also provides blocking solutions, reaction solutions and reagents for treating test samples, etc. as required.

An embodiment of a determination method for CRP using the CRP determination reagent of the present invention may include a determination method using an aggregation reaction typical of LPIA methods. "Determination" as used herein includes quantitative or non-quantitative determination. For example, non-quantitative determination may be a method of qualitative analysis wherein the degree of obtained aggregation is determined visually as either positive (+) or negative (-). Quantitative determination may be a method of determining the concentration of CRP or the amount of CRP.

The reaction between the CRP determination reagent and the test sample is an antigen-antibody reaction resulting in an aggregation reaction. There is no particular limitation on the conditions for the reaction as long as the reaction takes place. For example, a constant temperature of between 20 to 37°C for a period of between 5 seconds to 15 minutes is preferred.

The reaction between the CRP determination reagent and the test sample is generally performed in a buffer solution. There is no particular limitation on the buffer used as long as the antigen-antibody reaction occurs. For example, the following buffers are preferred: phosphate buffer solution, glycine buffer solution, Tris-HCL buffer solution and Good's buffer solution. The pH for the reaction is preferably between a pH of 7 to 9. In order to improve sensitivity, promote the reaction or stabilize the reaction, suitable amounts of the following may be added to the reaction solution: water-soluble polymers such as polyethylene glycol, polyvinylpyrrolidone and pullulan; stabilizers such as bovine serum albumin and sucrose; antiseptics such as sodium azide; and additives such as sodium chloride.

During the reaction between the CRP determination reagent and the antigen in the test sample, the concentration in the reaction solution of the insoluble carrier loading an anti-CRP antibody, for example when the insoluble carrier is a latex particle, is preferably between 0.01 to 5% (w/v). Concentrations of less than 0.01% (w/v) hardly provide the required sensitivity due to insufficient aggregate formation. Conversely, concentrations of more than 5% (w/v) result in excessive background absorbance which precludes accurate determination.

There is no particular limitation on the test sample as long as the sample can contain CRP. Examples of the test sample include blood, intercellular fluid, blood plasma, extravascular fluid, cerebrospinal fluid, synovial fluid, pleural fluid, serum, lymphatic fluid, saliva and urine. Furthermore the test sample of the present invention includes samples obtained from test subject, for example, a culture solution of cells from the body of a living organism.

When the degree of resulting aggregation is evaluated qualitatively, observation by the naked eye is sufficient. When determining the amount of aggregation quantitatively, the aggregrates may be determined using an optical method. There is no particular limitation on normally employed methods of optical determination and optical determination devices such as general spectrophotometers, biochemical autoanalyzers employing spectrophotometric determination principles, devices using near-infrared wavelength as a determination wavelength, devices using integrating-sphere turbidity determination principles and devices determining scattered light intensity may be used.
Methods determining the CRP concentration on the basis of variation in the absorbance per unit time during exposure of the resulting aggregates to near-infrared radiation are preferred. The determination wavelength is between 400 to 1000 nm and preferably between 500 to 800 nm. Quantitative determination of CRP is performed by preparing determining samples containing a known amount such as a reference serum and a corresponding dilution series. CRP is quantified using the CRP determination reagent of the present invention to prepare a calibration curve using the determined value and the known concentrations. Thus it is possible to work out an unknown amount in a sample using the pre-prepared calibration curve and the determined value under the same conditions.

### Examples

The present invention will be described in further detail hereafter with reference to the Examples, however the present invention is not limited to the Examples thereby.

The reagents and materials used in the Examples are described hereafter.
Latex particles; polystyrene latex ("Immutex", product of JSR Corporation) of average particle size 0.25 to 0.37 µm and having a carboxyl group modification to the surface were used.
Buffer to suspend latex, buffer for anti-CRP antibody dilution, anti-CRP antibodies, antigen CRP reference solution; reagents for CRP determination from Dako Ltd. were used.

### Example 1 Preparation of latex reagent (1)

2.0 mL of WSC solution at a concentration of 20 mg/mL and 0.23 mL of 50 mg/mL NHS solution were added sequentially while stirring to 1.0 mL of 1% suspension of latex particles modified with carboxyl groups. Stirring was continued for 30 minutes at 25°C and the carboxyl groups on the latex surface were activated. After activation, the suspension was washed with 0.05 M MES buffer (pH 6.5), 2 mL of any of spacer molecules (Arg, His, Lys, Glu, Asp, Gly, Ala, Ser, Thr, Cys, Asn, Gln, Leu, Ile, Val, Met, Phe, Tyr, Trp, Pro) at a concentration of 1.33 × 10⁻⁴ mol/mL in the same MES buffer was then added and stirring was continued at 37°C for 1 hour to bind the spacer molecules to the latex particles. 2 mL of the above WSC solution and 0.23 mL of the above NHS solution were added sequentially to the resulting solution, while stirring, to activate the carboxyl groups of the spacer molecules. After stirring, the solution was centrifuged (16000 rpm, 4°C, 20 minutes) to separate the precipitate from the supernatant. The precipitate was washed in the above MES buffer. 30 µL of 5.0 mg/mL anti-CRP antibody solution was added and stirred for 30 minutes at 37°C to bind the antibodies to the spacer molecules. After binding, the solution was centrifuged (16000 rpm, 4°C, 20 minutes) to separate the precipitate from the supernatant. The supernatant was used to quantify the amount of anti-CRP antibody binding to the latex molecule in a subsequent operation.
The precipitate was suspended in 2 mL of the above MES buffer solution in order to wash the particles. Once after binding, the solution was centrifuged (16000 rpm, 4°C, 20 minutes). After centrifuging, the precipitate was suspended in 1.0 mL of the same MES buffer solution, added to 1 mL of denatured BSA and stirred for 30 minutes at 25°C to block the binding sites of anti-CRP antibodies on the latex particle surface. After blocking, the mixture was suspended in 2.0 mL of 0.1 M Tris-HCl buffer solution (pH 8.2) to hydrolyze the unreacted activated carboxyl groups. After hydrolyzing, the particles were washed by suspending in 2.0 mL of the same Tris-HCl buffer solution and centrifuged (16000 rpm, 4°C, 20 minutes) twice. Latex reagent (1) is the resulting suspension in 2.0 mL of Tris-HCl buffer solution.

### Comparative Example 1 Preparation of latex reagent (2)

For the purposes of comparison, a latex reagent was prepared in which an anti-CRP antibody was loaded without the use of a spacer molecule.
2.0 mL of WSC solution at a concentration of 20 mg/mL and 0.23 mL of 50 mg/mL NHS solution were added sequentially while stirring to 1.0 mL of 1% suspension of latex particles modified with carboxyl groups. Stirring was continued at 25°C for 30 minutes to activate the carboxyl groups on the latex surface. 30 µL of 5.0 mg/mL anti-CRP antibody solution was added and stirred for 30 minutes at 37°C to bind the antibodies to the latex particles. After binding, the solution was centrifuged (16000 rpm, 4°C, 20 minutes) to separate the precipitate from the supernatant. The supernatant was used to quantify the amount of anti-CRP antibody binding to the latex particle in a subsequent operation.
The precipitate was suspended in 2.0 mL of the above MES buffer solution and added to 1.0 mL of 1.5% denatured BSA and stirred for 30 minutes at 25°C to block the binding sites of anti-CRP antibodies on the latex particle surface. After blocking, the mixture was suspended in 2.0 mL of 0.1 M Tris-HCl buffer solution (pH 8.2) to hydrolyze the unreacted activated carboxyl groups. Latex reagent (2) is the resulting suspension in 2.0 mL of Tris-HCl buffer solution.

### Comparative Example 2 Preparation of latex reagent (3)

Latex reagent (3) was prepared in the same manner as Example 1 with the exception of binding protein A as a spacer molecule to the latex particle by using a solution of 0.5 mg of protein A (product of SIGMA Co.) dissolved in 1 mL of 0.05 M MES buffer (pH 6.5).

### Test Example 1 Determination of the amount of anti-CRP antibody binding to the latex particle

A BCA method was used to determine the amount of anti-CRP antibody binding to the latex particle using each supernatant obtained in Example 1 or Comparative Example 1 above. In other words, a sample solution was mixed into a solution of bicinchoninic acid and the binding amount was calculated using absorbance at a wavelength of 562 nm.
The binding amount in each latex reagent (1) was confirmed as being approximately 65 to 75% on average with respect to the amount of added antibody. The binding amount in latex reagent (2) was approximately 70% of the amount of added antibody.

### Test Example 2 Evaluation of the latex reagents using a standard solution

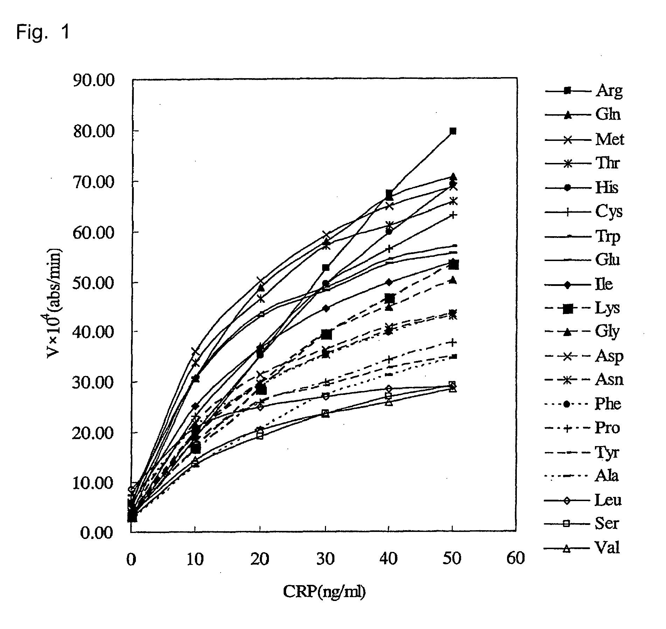

Latex reagents (1) and (2) prepared in Example 1 and Comparative Example 1 were respectively reacted with CRP in order to determine the aggregration reaction rate using a Latex Photometric Immunoassay (LPIA Method). Determinations were performed using a fully-automatic immunoserological test system LPIA-200 (product of Diayatron Co., Ltd.). In other words, 30 µL of antigen CRP standard solution, 230 µL of Tris-HCl-BSA buffer solution (pH 8.2) and 30 µL of each latex reagent were dispensed into a determining cuvette. The change of absorbance was determined at a wavelength of 950 nm at 12-second intervals for 10 min. Determination of the detection limit and the amount of CRP was performed by preparing a calibration curve from the average reaction rate of the latex reagents for different spacers. The results of the reactivity for each latex reagent in Example 1 are shown in Figure 1.
The results show that each latex reagent (1) from Example 1 results in stable and highly sensitive determinations until CRP antigen concentrations of 10 ng/mL. On the other hand, the determinations using latex reagent (2) from Comparative Example 1 were limited to 500 ng/mL. Consequently the reagent which binds an antibody to the surface of the latex using an amino acid as a spacer molecule was confirmed to be able to determine CRP at extremely high sensitivities. The spacer molecules displayed excellent sensitivity in the order of Arg > Gln > Met > Thr > His > Cys > Trp > Glu > Ile > Lys > Gly > Asp > Asn > Phe > Pro > Tyr > Ala > Leu > Val > Ser. The difference in reactivity between Thr and Ser which were both oxyamino acids was thought to result from the effect on reactivity of the difference in the position of the hydroxy group. Furthermore the difference between Met and Cys which were both sulfur-containing amino acids was thought to result from the effect on reactivity of the length of side chains and the property of the spacer itself.

### Test Example 3 Evaluation of the latex reagents using a standard solution

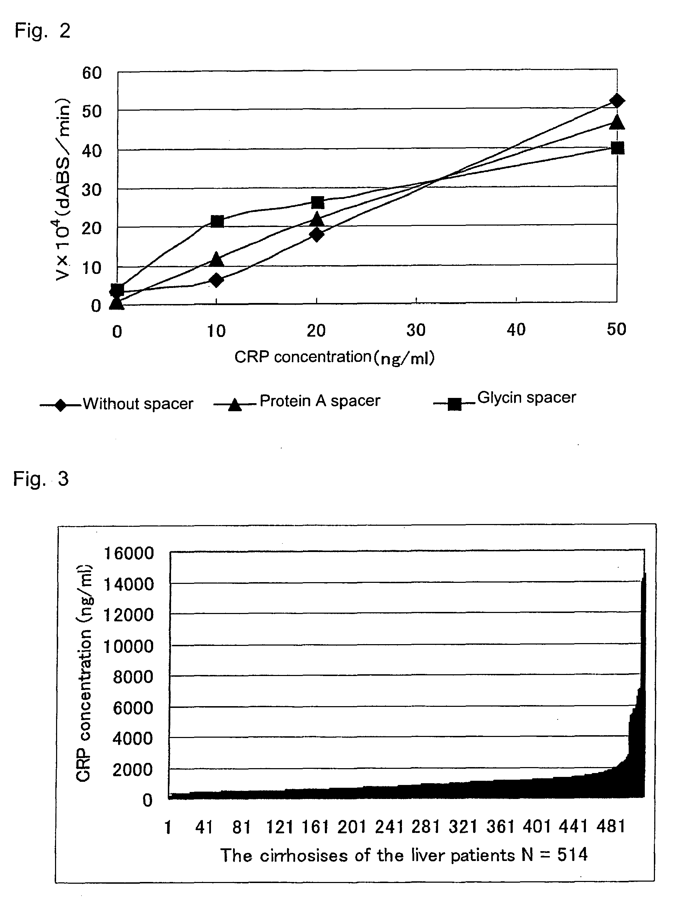

Latex reagent (1) prepared in Example 1 using glycine (Gly) as the spacer molecule together with latex reagents (2) and (3) prepared in Comparative Examples 1 and 2 were respectively reacted with CRP. In the same manner as Test Example 2, the aggregation reaction rates were determined using a Latex Photometric Immunoassay (LPIA Method). The reactivity results for each latex reagent are shown in Figure 2.
The results show that latex reagent (1) using glycine (Gly) as the spacer molecule results in stable determinations with about five times the sensitivity at low concentration ranges as latex reagents (2) and (3) prepared in Comparative Examples 1 and 2.

### Test Example 4 Determination of CRP in human serum

The CRP concentration in serum was determined using 0.5 µL of serum obtained from a healthy subject or a patient suffering from age-related (senile) macular degeneration instead of the antigen CRP standard solution used in Test Example 2.

Using latex reagent (1) obtained in Example 1, the results show that the concentration of CRP antigen in the healthy subject was an average of 422 ng/mL for 30 samples (maximum 1825 ng/mL, minimum 199 ng/mL) and in the patient suffering from age-related macular degeneration was an average of 867 ng/mL for 32 samples (maximum 2694 ng/mL, minimum 327 ng/mL). The results show that the patient suffering from age-related macular degeneration could be distinguished from the normal subject. Thus the determination reagent of the present invention can be applied even to extremely small internal inflammations.

### Test Example 5 Determination of CRP in blood of patient suffering from hepatic disease

The CRP concentration in blood was determined using 0.5 µL of blood obtained from patients suffering from various liver diseases instead of the antigen CRP standard solution used in Test Example 2. The latex reagent using glycine (Gly) as a spacer molecule was employed. The results are shown in Figure 3.
The results show that the concentration of CRP antigen was an average of 1,106 ng/mL for 514 samples, maximum 14,419 ng/mL, minimum 157 ng/mL. Since CRP is produced in the liver, a reduction in capacity for CRP production is suggested in hepatic disease. However high values for CRP resulting from disease were confirmed using the latex reagent of the present invention. These data show clearly that CRP is also produced as a result of inflammation of the liver in patients suffering from hepatic disease. Thus determination of the CRP concentration in serum using the determination reagent of the present invention can be expected to be useful for monitoring the complications or course of treatment in patients suffering from hepatic disease, etc.

## Claims

1. A C-reactive protein determination reagent comprising an insoluble carrier particle loading an antibody directed to the C-reactive protein via an amino acid.

2. The C-reactive protein determination reagent according to claim 1, wherein the amino acid is selected from Gly, Ala, Ser, Thr, Cys, Asn, Gln, Leu, Ile, Val, Met, Phe, Tyr, Trp, Pro, Asp, Glu, His, Lys and Arg, a salt thereof and a hydrate thereof.

3. The C-reactive protein determination reagent according to claim 1 or 2, wherein the insoluble carrier particle is a polystyrene latex particle modified with a carboxyl group.

4. A method of determining a C-reactive protein comprising the steps of reacting the C-reactive protein determination reagent according to any one of claims 1 to 3 with an antigenic substance in a test sample, and determining the resulting aggregate.

5. The determination method according to Claim 4 wherein the test sample is blood, serum or plasma.

6. The determination method according to claim 4 or 5, comprising the step of quantifying a concentration of a C-reactive protein from a change obtained in absorbance per unit time during exposure of the resulting aggregate to near-infrared radiation.
